# EUROPÄISCHE PATENTANMELDUNG

(11) **EP 2 878 341 A2**
(43) Veröffentlichungstag der Anmeldung: **03.06.2015**
(21) Anmeldenummer: 14158135.5
(22) Anmeldetag: 06.03.2014
(51) Int. Cl.: A61Q 19/00, A61K 8/97

(54) **Verwendung kosmetischer Zusammensetzungen für die Inaktivierung und/oder Eliminierung von Hautmilben**

(30) Priorität: 21.11.2013 DE 102013223790
(71) Anmelder: Henkel AG & Co. KGaA, 40589 Düsseldorf (DE)
(72) Erfinder: Kessler-Becker, Daniela, 51371 Leverkusen (DE); Riecke, Clemens, 425853 Remscheid (DE)

(57) **Zusammenfassung**

Gegenstand der Erfindung ist die kosmetische Verwendung einer kosmetischen Zusammensetzung, die mindestens einen wässrigen oder wässrig-alkoholischen Pflanzenextrakt enthält, für die Inaktivierung und/oder Eliminierung von Milben auf der Haut.

Gegenstand der Erfindung ist weiterhin ein kosmetisches, nichttherapeutisches Verfahren zur optischen Verbesserung und/oder Erhaltung des Erscheinungsbildes menschlicher Haut, bei dem zur Inaktivierung und/oder Eliminierung von Milben eine kosmetische Zusammensetzung, enthaltend mindestens einen wässrigen oder wässrig-alkoholischen Pflanzenextrakt, auf die Haut aufgetragen und nach einer Einwirkungszeit von 30 Sekunden bis 24 Stunden gegebenenfalls mit Wasser wieder abgespült wird.

## Beschreibung

Die vorliegende Erfindung liegt auf dem Gebiet der Kosmetik und betrifft die Verwendung kosmetischer Zusammensetzungen, die mindestens einen wässrigen oder wässrig-alkoholischen Pflanzenextrakt enthalten, für die Inaktivierung und/oder Eliminierung von Milben auf der Haut.
Die Erfindung betrifft weiterhin ein kosmetisches, nichttherapeutisches Verfahren zur optischen Verbesserung und/oder Erhaltung des Erscheinungsbildes menschlicher Haut.

Das natürliche Erscheinungsbild der menschlichen Haut und/oder Kopfhaut kann sich durch dermatologische Erkrankungen, Umwelteinflüsse (Sonne, Wind, Heizungen, Klimageräte etc.) sowie durch den normalen Alterungsprozess im Laufe des Lebens stark verändern.
Es ist eine Vielzahl kosmetischer Präparate bzw. kosmetischer Behandlungsprozesse bekannt, mit denen Zeichen der Hautalterung und/oder die Folgen von Umwelteinflüssen auf der Haut (trockene Haut, Sonnenbrand, Pigmentflecken etc.) behandelt werden können.
Problematischer ist die kosmetische Behandlung von Hauterkrankungen wie beispielsweise Rosacea und/oder Akne vulgaris, denn auch die oftmals erfolgreiche kosmetische Behandlung der Symptome dieser Erkrankungen führt nicht immer dazu, dass sie nachhaltig beseitigt bzw. gelindert werden können.
Seit vielen Jahren ist man daher bemüht die Ursachen, Auslöser und/oder die begünstigenden Faktoren von Hauterkrankungen möglichst vollständig aufzuklären, um kosmetische Präparate zu entwickeln, die nicht nur Hauterkrankungen lindern oder beseitigen, sondern auch vorbeugend gegen Hauterkrankungen wirken.
So ist bekannt, dass bestimmte Milbenarten die menschliche Haut, vorzugsweise die Gesichtshaut, die Kopfhaut und/oder die Haut im Brust- oder Kniekehlenbereich, besiedeln.
Allein die Besiedelung der Haut mit den Milben scheint unproblematisch zu sein.
Es wurde jedoch in einigen Fällen gefunden, dass bei Auftreten bestimmter Hautveränderungen (so genannter Demodikosen, die optisch an Rosacea oder Akne vulgaris erinnern) ein besonders großer Befall der Haut mit Milben nachgewiesen werden konnte.

Fortan stand die Entwicklung kosmetischer Mittel im Fokus, die die Haut vor der Besiedelung mit Milben schützen oder die die bereits vorhandenen Hautmilben inaktivieren oder eliminieren. Ziel dieser Entwicklungen war vor allem die übermäßige Vermehrung von Hautmilben zu verhindern und damit Hauterkrankungen vorzubeugen.

In der Vergangenheit wurden mehrere kosmetische Methoden zur Hautbehandlung vorgeschlagen, die insbesondere der Bekämpfung von Hautmilben dienten. So wurden neben speziellen Öl(en)(mischungen) (KR 2011/0026879, EP 2192913) auch spezielle Wirkstoffe wie beispielsweise Benzoylperoxid (EP 1570850) und Ciclopirox (EP 1682083) verwendet.

Die Verwendung stark ölhaltiger kosmetischer Präparate oder die Verwendung hautreizender Wirkstoffe ist aber nicht immer wünschenswert, denn sie können die Haut in anderer Art und Weise negativ beeinflussen und andere unerwünschte Veränderungen der Haut (wie beispielsweise fettig glänzende Haut; Pickel; trockene, schuppige und/oder gerötete Haut) zur Folge haben oder begünstigen.

Ziel der vorliegenden Erfindung war es daher kosmetische Wirkstoffe für die problemlose Einarbeitung in kosmetische Mittel zu finden, die Hautmilben wirksam bekämpfen, und die gleichzeitig keine (oder möglichst wenige) anderen unerwünschten negativen Reaktionen auf der Haut hervorrufen. Optimalerweise sollten Wirkstoffe auf natürlicher Basis gefunden werden, die das Gleichgewicht der Haut nicht belasten.

Es wurde nun gefunden, dass der Einsatz wässriger oder wässrig-alkoholischer Pflanzenextrakte in kosmetischen Hautbehandlungsmitteln dazu führt, dass Hautmilben wirksam inaktiviert bzw. eliminiert werden können.

Ein erster Gegenstand der vorliegenden Erfindung ist somit die kosmetische Verwendung einer mindestens einen wässrigen oder wässrig-alkoholischen Pflanzenextrakt enthaltenden kosmetischen Zusammensetzung für die Inaktivierung und/oder Eliminierung von Milben auf der Haut.

Unter "Haut" wird erfindungsgemäß gesunde menschliche Haut verstanden, bevorzugt die üblicherweise stärker von Milbenbefall betroffenen Hautpartien des menschlichen Körpers wie die Gesichtshaut (vorzugsweise der Stirn-, Kinn-, Wangen- und Augenlidbereich der Gesichtshaut), die Kopfhaut, die Haut im Brust-, Hals- und Dekolleteebereich oder die Haut im Achsel-, Armbeugen- oder Kniekehlenbereich.
Insbesondere geeignet ist die erfindungsgemäße Verwendung für die Inaktivierung und/oder Eliminierung von Milben auf der menschlichen Gesichts- und/oder Kopfhaut.

Unter "Milben" werden erfindungsgemäß bevorzugt Milben aus der Gattung der Haarbalgmilben *(Demodex)* verstanden.
Demodex-Milben gehören zu den normalen Kommensalen der menschlichen Haut. Sie leben in den Talgdrüsenausführungsgängen sowie rund um die Haarfollikel und sind geschlechtsunabhängig und wirtsspezifisch bei Menschen aller Hautfarben und Herkunft weit verbreitet. Sie besiedeln annähernd jeden Menschen im Laufe seines Lebens. Die Übertragung erfolgt von Mensch zu Mensch und der Befall beginnt schon bei Säuglingen mit dem ersten Mutter-Kind-Kontakt. Mit zunehmendem Alter nimmt die Dichte der Milben pro Follikel zu: während Neugeborene noch unbefallen sind, sind über 70-Jährige zu 100% befallen.
Auf der menschlichen Haut konnten zwei Arten von Demodex-Milben nachgewiesen werden, die sogenannten *Demodex brevis* und die *Demodex folliculorum.*

Meist sind die Milben harmlos, jedoch können sie auch entzündliche Talgdrüsenerkrankungen (Demodikosen) hervorrufen. Typisch für eine Demodikose sind streng einseitige, randbetonte, erythrematöse und papulopustulöse Läsionen, die vorzugsweise an den Wangen, dem Kinn oder der Stirn auftreten.

Die auslösenden Faktoren der Demodikose sind nicht vollends bekannt. Es besteht aber die begründete Vermutung, dass ein höheres Alter und/oder ein schlechter Allgemeinzustand den übermäßig starken Befall der Haut mit Demodex-Milben begünstigen können.
Bei besonders hoher Milbendichte können dann - eventuell ausgelöst durch den Kot oder die Eier der Milben - die zuvor genannten Hautveränderungen auftreten.

Die erfindungsgemäße Verwendung kosmetischer Mittel, die mindestens einen wässrigen oder wässrig-alkoholischen Pflanzenextrakt enthalten, ist prinzipiell für die Inaktivierung und/oder Eliminierung beider Demodex-Milbenarten geeignet. Besonders gute Ergebnisse konnten aber bei der Bekämpfung von Milben der Art *Demodex folliculorum* erzielt werden.
In einer ersten bevorzugten Ausführungsform ist die erfindungsgemäße Verwendung demnach gerichtet auf die Inaktivierung und/oder Eliminierung von Milben der Art *Demodex folliculorum.*

Unter "Inaktivierung" wird verstanden, dass die Milben durch die erfindungsgemäße Verwendung so stark geschädigt werden, dass sie bevorzugt innerhalb von maximal einem Tag zu mindestens 10% unbeweglich werden.

Unter "Eliminierung" wird verstanden, dass die Milben durch die erfindungsgemäße Verwendung so stark geschädigt werden, dass sie innerhalb von maximal 3 Tagen, mehr bevorzugt innerhalb von maximal 2 Tagen und insbesondere innerhalb von maximal einem Tag zu mindestens 10%, bevorzugt zu mindestens 20% und insbesondere zu mindestens 30% sterben.

Unter geeigneten Pflanzenextrakten sind Extrakte zu verstehen, die aus allen Teilen einer Pflanze hergestellt werden können.
Üblicherweise werden diese Extrakte durch Extraktion der gesamten Pflanze hergestellt. Es kann aber in einzelnen Fällen auch bevorzugt sein, die Extrakte ausschließlich aus den Blüten, den Blättern, den Früchten, der Rinde oder der Wurzel der Pflanze herzustellen.
Beispiele für Pflanzenextrakte, die erfindungsgemäß verwendet werden können, sind bevorzugt ausgewählt aus Grünem Tee, Eichenrinde, Großwiesenknopf, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwer, Echinacea purpurea, Olea europea, Foeniculum vulgaris und Apim graveolens.

Als Extraktionsmittel zur Herstellung der genannten Pflanzenextrakte können Wasser, Alkohole sowie deren Mischungen verwendet werden.
Als geeignete Alkohole werden bevorzugt niedere Alkohole wie Ethanol, Ethylenglycol, n-Propanol, Isopropanol, 1,2-Propandiol, 1-Butanol, 2-Butanol, Isobutanol, 1,2-Butylenglycol und/oder 1,3-Butylenglycol verwendet. Insbesondere geeignet sind mehrwertige Alkohole wie Ethylenglykol, Propylenglykol und Butylenglycol, sowohl als alleiniges Extraktionsmittel als auch in Mischung mit Wasser. Pflanzenextrakte auf Basis von Wasser oder von Wasser/Butylenglykol im Verhältnis 1:10 bis 10:1 haben sich als besonders geeignet erwiesen.
Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Für die erfindungsgemäße Verwendung haben sich bestimmte Pflanzenextrakte als besonders wirksam gegen Demodex-Milben herausgestellt.
So wurde gefunden, dass insbesondere Pflanzenextrakte aus der Familie der Ingwergewächse *(Zingiberaceae),* Pflanzenextrakte aus der Familie der Rosengewächse (Rosaceae) und/oder Pflanzenextrakte aus der Familie der Lorbeergewächse *(Lauraceae)* effektiv für die Bekämpfung von Demodex-Milben verwendet werden können.

Eine Kombination von Pflanzenextrakten aus allen (drei) zuvor genannten Familien führte zu besonders zufrieden stellenden Ergebnissen.

In einer ersten bevorzugten Ausführungsform enthalten für die erfindungsgemäße Verwendung geeignete kosmetische Mittel daher eine Mischung aus
- mindestens einem wässrigen oder wässrig-alkoholischen Pflanzenextrakt aus der Familie der Ingwergewächse und/oder
- mindestens einem wässrigen oder wässrig-alkoholischen Pflanzenextrakt aus der Familie der Lorbeergewächse und/oder
- mindestens einem wässrigen oder wässrig-alkoholischen Pflanzenextrakt aus der Familie der Rosengewächse.

Innerhalb dieser Ausführungsform sind die wässrigen oder wässrig-alkoholischen Extrakte aus den Wurzeln und/oder der Rinden der zuvor genannten Pflanzenfamilien besonders geeignet.

Für die erfindungsgemäße Verwendung geeignete kosmetische Mittel dieser ersten Ausführungsform enthalten insbesondere bevorzugt eine Mischung aus
- mindestens einem wässrigen oder wässrig-alkoholischen Extrakt aus den Wurzeln von *Zingiber officinalis,*
- mindestens einem wässrigen oder wässrig-alkoholischen Extrakt aus der Rinde von *Cinnamomum Cassia,* und
- mindestens einem wässrigen oder wässrig-alkoholischen Pflanzenextrakt aus den Wurzeln von *Sanguisorba officinalis.*

Die einzelnen Pflanzenextrakte sind im Handel erhältlich, beispielsweise wässrige Rindenextrakte aus der Rinde von *Cinnamomum Cassia,* wässrige Extrakte aus den Wurzeln von *Zingiber officinalis* und wässrig-alkoholische (Wasser/Butylenglycol) Extrakte aus den Wurzeln von *Sanguisorba officinalis.* Unter der Handelsbezeichnung "Sebustop^{®}" ist im Handel von der Firma Solabia auch eine gebrauchsfertige Mischung der zuvor genannten Pflanzenextrakte erhältlich (INCI: Butylene Glycol, Water, Poterium (Sanguisorba) officinale root extract, Cinnamonum cassia bark extract and Zingiber officinale root extract). Sie weist Anteile der Einzelbestandteile im Bereich von jeweils 1-40 Gew-% auf, bezogen auf das Trockengewicht der eingesetzten Rohstoffe. Der Extrakt enthält essentielle Öle, Saponoside, Triterpene und Tannine. Er weist einen pH-Wert im Bereich von 4,0 bis 6,0 auf und ist mischbar mit Wasser, Propylenglycol und mit 50 %igem Ethanol (nicht mischbar mit Mineralölen).

Der oder die wässrige(n) oder wässrig-alkoholische(n) Pflanzenextrakt(e) können in den erfindungsgemäß zu verwendenden kosmetischen Zusammensetzungen bevorzugt in Mengen von 0,1 bis 40 Gew.-%, bevorzugt von 1 bis 20 Gew.-% und insbesondere von 1,5 bis 5 Gew.-% enthalten sein, wobei sich die Mengen auf das Gesamtgewicht der kosmetischen Zusammensetzung beziehen.

Für die erfindungsgemäße Verwendung geeignet sind sowohl kosmetische Zusammensetzungen, die nach ihrer Anwendung und einer kurzen Einwirkungszeit (etwa 10 Sekunden bis zu einer Stunde) mit Wasser von der Haut wieder abgespült werden (so genannte rinse-off-Produkte), als auch kosmetische Zusammensetzungen, die bevorzugt nach der Reinigung auf die Haut aufgetragen werden und bis zur nächsten Reinigung darauf verbleiben (so genannte leave-on-Produkte).
Eine längere Einwirkungszeit der Zusammensetzungen kann jedoch für die Inaktivierung/Eliminierung der Demodex-Milben von Vorteil sein, deshalb sind kosmetische Zusammensetzungen bevorzugt, die auf der Haut verbleiben.

In einer zweiten bevorzugten Ausführungsform der Erfindung werden daher kosmetische Zusammensetzungen erfindungsgemäß verwendet, die auf der Haut verbleiben.

In einer dritten bevorzugten Ausführungsform der Erfindung werden in einem ersten Schritt zunächst kosmetische Zusammensetzungen erfindungsgemäß verwendet, die nach einer Einwirkungszeit von etwa 10 Sekunden bis zu einer Stunde mit Wasser von der Haut wieder abgespült werden, und in einem zweiten Schritt werden kosmetische Zusammensetzungen erfindungsgemäß verwendet, die auf der Haut verbleiben.

Erfindungsgemäß zu verwendende kosmetische Zubereitungen können in verschiedenen Angebotsformen konfektioniert werden, beispielsweise als (ggf. öl- und fettfreies) Gel, als Creme, in Stiftform, als flüssige oder gelförmige Roll-on-Applikation, als getränktes flexibles Substrat (Pad), aber auch als Puder oder Spray.

Erfindungsgemäß zu verwendende kosmetische Zubereitungen können in fester, halbfester, flüssiger, disperser, emulgierter, suspendierter, oder gelförmiger Form vorliegen. Weiterhin können sie als Aerosol konfektioniert sein, das heißt, sie sind in einem Druckbehälter verpackt, aus dem sie mit Hilfe eines Treibmittels versprüht werden können. Weiterhin können die kosmetischen Zubereitungen als treibgasfreies Pumpspray versprüht werden.

Bevorzugte erfindungsgemäß zu verwendende kosmetische Zubereitungen können beispielsweise Reinigungsmittel für die Haut wie Dusch- und Waschgele und/oder Seifen, Hautpflegemittel wie Lotionen, Gele und/oder Cremes für den Körper und/oder das Gesicht, Gesichtswässer, Lippenpflegemittel, aber auch dekorative kosmetische Mittel wie Makeups sein.
Besonders bevorzugte kosmetische Zubereitungen sind dadurch gekennzeichnet, dass sie als Hautpflegemittel, insbesondere als Hautpflegemittel für die Gesichtshaut, wie eine Gesichtscreme, ein Gesichtsgel und/oder ein Gesichtswasser, konfektioniert sind.

In einer besonders bevorzugten Ausführungsform enthalten erfindungsgemäß zu verwendende Mittel zur Erzielung verbesserter Pflegeeffekte auf der Haut zusätzlich zu dem oder den wässrigen oder wässrig-alkoholischen Pflanzenextrakt(en) bevorzugt einen geeigneten kosmetischen Träger enthalten, vorzugsweise einen topischen Träger, der weiterhin mindestens einen Wirkstoff, ausgewählt aus
- Harnstoff-Derivaten, ausgewählt aus Verbindungen gemäß Formel (I) 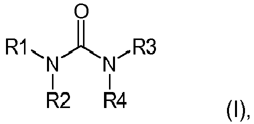 worin
   die Reste R1, R2, R3 und R4 unabhängig voneinander für ein Wasserstoffatom, eine C₁-C₄-Alkylgruppe, eine C₂-C₆-Alkenylgruppe oder eine C₂-C₆-Hydroxyalkylgruppe mit mindestens einer Hydroxygruppe steht,
   mit der Maßgabe, dass mindestens einer der besagten Reste eine C₂-C₆Hydroxyalkylgruppe mit mindestens einer Hydroxygruppe bedeutet,
- Vitaminen, Provitaminen und Vitaminvorstufen der Gruppen B, C, E, H und K und den Estern der vorgenannten Substanzen,
- Monomeren, Oligomeren und Polymeren von Aminosäuren, N-C₂-C₂₄-Acylaminosäuren, den Estern und/oder den physiologisch verträglichen Metallsalzen dieser Substanzen,
- DNA- oder RNA-Oligonucleotiden,
- natürlichen Betainverbindungen,
- α-Hydroxycarbonsäuren, α-Ketocarbonsäuren, β-Hydroxycarbonsäuren und deren Ester-, Lacton-oder Salzform,
- den Flavonoiden Naringin, α-Glucosylrutin, α-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid,
- Isoflavonoiden und Isoflavonoid-reichen Pflanzenextrakten,
- Ubichinon und Ubichinol sowie deren Derivaten,
- Silymarin,
- natürlich vorkommenden Xanthin-Derivaten, ausgewählt aus Coffein, Theophyllin, Theobromin und Aminophyllin,
- Ectoin,
- Kreatin,
- Olivenblattextrakten, Ursolsäure, Oleanol und/oder Oleanolsäure,
- Mono- und Polyhydroxystilbenen und deren Estern,
- Derivaten von methyliertem Silanol,
- Phytinsäure,
- Extrakten aus Maiskörnern (Zea Mays (Corn) Kernel Extract),
- Extrakten aus Haferkörnern (Avena Sativa (Oat) Kernel Extract),
- Saccharomyces/Xylinum/Black Tea Ferment,
- Apfelkernextrakten (Pyrus Malus (Apple) Fruit Extract),
- Lotuskeim-Extrakten (Nelumbo Nucifera Germ Extract),
- Rotweinextrakten,
- Traubenkernextrakten (Vitis Vinifera (Grape) Seed Extract), die bevorzugt aus der Chardonnay-Traube stammen,
- Extrakten aus Schwarzen Holunderblüten (Sambucus Nigra Flower Extract),
- Wirkstoffen, die die beta-Endorphinsynthese in Keratinozyten stimulieren,
- anorganischen und organischen UV-Filtersubstanzen,
- hautberuhigenden Wirkstoffen,
- Wirkstoffen, die die Prostaglandinsynthese und/oder die Leukotrien-Synthese inhibieren,
- sebumregulierenden Wirkstoffen,
- sowie Desoxyzuckern oder Desoxyzucker-Bausteine enthaltenden Polysacchariden, enthält.

Unter einem geeigneten kosmetischen Träger, bevorzugt einem topischen Träger, wird bevorzugt ein wässriger oder wässrig-alkoholischer Träger verstanden.
Bevorzugt enthält der Träger - bezogen auf sein Gesamtgewicht - mindestens 30 Gew.-%, mehr bevorzugt mindestens 35 Gew.-% besonders bevorzugt mindestens 40 Gew.-% und insbesondere mindestens 45 Gew.-% Wasser.
Weiterhin kann der kosmetische Träger 0,01 bis 40 Gew.-%, bevorzugt 0,05 bis 35 Gew.-% und insbesondere 0,1 bis 30 Gew.-% mindestens eines Alkohols enthalten, der ausgewählt sein kann aus Ethanol, 1-Propanol, 2-Propanol, Isopropanol, Glycerin, Diglycerin, Triglycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycole, Sorbitol, Sorbitan, Benzylalkohol, Phenoxyethanol oder Mischungen dieser Alkohole.
Bevorzugt sind die wasserlöslichen Alkohole.

Besonders bevorzugt sind Ethanol, 1-Propanol, 2-Propanol, 1,2-Propylenglycol, Glycerin, und/oder 1,6-Hexandiol sowie Mischungen dieser Alkohole. Insbesondere bevorzugt sind Glycerin und/oder 1,6-Hexandiol.

Besonders bevorzugte Harnstoffderivate der Formel (I) sind solche, die mindestens eine C₂-C₆-Hydroxyalkylgruppe mit mindestens einer Hydroxygruppe enthalten, welche aus mindestens einem Vertreter aus der Gruppe aus 2-Hydroxyethyl, 2-Hydroxy-2-methylprop-2-yl, 1,3-Dihydroxy-2-methylprop-2-yl, 1,3-Dihydroxyprop-2-yl, Tris(hydroxymethyl)methyl, 1,3-Dihydroxy-2-hydroxymethylprop-2-yl, 2,3-Dihydroxypropyl, 2-Hydroxypropyl, 3-Hydroxypropyl, 4-Hydroxybutyl, 1-Hydroxy-2-methylprop-2-yl, 2,3,4,5,6-Pentahydroxyhexyl und 1,3,4,5,6-Pentahydroxyhex-2-yl ausgewählt wird.

Es ist bevorzugt solche Harnstoffderivate der Formel (I) zu verwenden, welche die bevorzugte Maßgabe der Formel (I)erfüllen, dass mindestens einer der Reste R1 bis R4 eine C₂-C₆-Hydroxyalkylgruppe mit mindestens einer Hydroxygruppe bedeutet. Weiterhin besonders bevorzugt stehen jeweils zwei Reste aus R1, R2, R3 und R4 für eine C₂-C₆-Hydroxyalkylgruppe mit mindestens einer Hydroxygruppe. Insbesondere bevorzugt stehen die zwei Reste aus R1, R2, R3 und R4 für eine C₂-C₆-Hydroxyalkylgruppe mit mindestens einer Hydroxygruppe, wobei diese beiden Reste an unterschiedlichen Stickstoffatomen des Harnstoffs positioniert (N,N'-Stellung) sind. Beispiele für in Verbindungen der Formel (I)verwendbare C₁-C₄-Alkylgruppen sind Methyl, Ethyl, n-Propyl, Isopropyl, n-Butyl, sec-Butyl, Isobutyl und tert-Butyl. Beispiele für in Verbindungen der Formel (I) verwendbare C₂-C₆-Alkenylgruppen sind Vinyl, 2-Propen-1-yl (Allyl) oder 3-Buten-1-yl.

Besonders bevorzugt wird mindestens eine Verbindung aus der Gruppe mit den Vertretern N-(2-Hydroxyethyl)harnstoff, N,N-Bis-(2-hydroxyethyl)harnstoff, N,N'-Bis-(2-hydroxyethyl)harnstoff, N-(3-Hydroxypropyl)harnstoff, N,N-Bis-(3-hydroxypropyl)harnstoff, N,N'-Bis-(3-hydroxypropyl)harnstoff, N-(2-Hydroxypropyl)harnstoff, N,N-Bis-(2-hydroxypropyl)harnstoff, N,N'-Bis-(2-hydroxypropyl)harnstoff, N-(2-Hydroxy-2-methyl-prop-2-yl)harnstoff, N,N-Bis-(2-hydroxy-2-methyl-prop-2-yl)harnstoff, N,N'-Bis-(2-hydroxy-2-methyl-prop-2-yl)harnstoff, N-(1,3-Dihydroxy-2-methyl-prop-2-yl)harnstoff, N,N-Bis-(1,3-dihydroxy-2-methyl-prop-2-yl)harnstoff, N,N'-Bis-(1,3-dihydroxy-2-methyl-prop-2-yl)harnstoff, N-(1,3-Dihydroxy-2-hydroxymethyl-prop-2-yl)harnstoff, N,N-Bis-(1,3-dihydroxy-2-hydroxymethyl-prop-2-yl)harnstoff und N,N'-Bis-(1,3-dihydroxy-2-hydroxymethyl-prop-2-yl)harnstoff ausgewählt. Ganz besonders bevorzugt ist N-(2-Hydroxyethyl)harnstoff und N,N'-Bis-(2-hydroxyethyl)harnstoff, erhältlich beispielsweise als Handelsprodukt Hydrovance^{®} von der Firma National Starch.

Erfindungsgemäß zu verwendende kosmetische Mittel enthalten Harnstoff-Derivate der zuvor genannten Formel (I) bevorzugt in Mengen von 0,75 bis 4,5 Gew.-%, mehr bevorzugt von 1 bis 4 Gew.-%, besonders bevorzugt von 2 bis 3,5 Gew.-% und insbesondere von 2,5 bis 3,2 Gew.-%, wobei sich die Mengenangaben auf das Gesamtgewicht des Mittels beziehen.

Geeignete Vitamine, Provitamine oder Vitaminvorstufen der Vitamingruppen B, C, E, H und K können beispielsweise ausgewählt sein aus:
(i) Vitamin B: Zur Vitamin B-Gruppe oder zu dem Vitamin B-Komplex gehören unter anderem
   - Vitamin B₁: Trivialname Thiamin, chemische Bezeichnung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in den erfindungsgemäß verwendeten Mitteln in Mengen von 0,05 bis 1 Gew.-%, bezogen auf das gesamte Mittel, eingesetzt,
   - Vitamin B₂: Trivialname Riboflavin, chemische Bezeichnung 7,8-Dimethyl-10-(1- D-ribityl)-benzo[g]pteridin-2,4(3H,10H)-dion. Bevorzugt werden Riboflavin oder seine Derivate in den erfindungsgemäß verwendeten Mitteln in Mengen von 0,05 bis 1 Gew.- %, bezogen auf das gesamte Mittel, eingesetzt,
   - Vitamin B₃: Unter dieser Bezeichnung werden die Verbindungen Nicotinsäure und Nicotinsäureamid (Niacinamid) geführt. Bevorzugt ist das Nicotinsäureamid, das in den erfindungsgemäß verwendeten Mitteln bevorzugt in Mengen von 0,05 bis 1 Gew.- %, bezogen auf das gesamte Mittel, enthalten sein kann,
   - Vitamin B₅: Pantothensäure, Panthenol und Pantolacton (Dihydro-3-hydroxy-4,4-dimethyl-2(3H)-furanon). Einsetzbare Derivate des Panthenols sind insbesondere die Ester und Ether des Panthenols sowie kationisch derivatisierte Panthenole.
      Die genannten Verbindungen des Vitamin B₅-Typs sowie die 2-Furanonderivate können in den erfindungsgemäß zu verwendenden Mitteln in einer Gesamtmenge von 0,05 bis 5 Gew.-%, bevorzugt von 0,1 bis 3 Gew.-%, besonders bevorzugt von 0,5 bis 2 Gew.-%, jeweils bezogen auf das gesamte Mittel, enthalten sein,
   - Vitamin B₆: hierunter wird keine einheitliche Substanz, sondern die unter den Trivialnamen Pyridoxin, Pyridoxamin und Pyridoxal bekannten Derivate des 5-Hydroxymethyl-2-methylpyridin-3-ols verstanden. Vitamin B₆ kann in den erfindungsgemäß zu verwendenden Mitteln bevorzugt in Mengen von 0,0001 bis 1,0 Gew.- %, insbesondere in Mengen von 0,001 bis 0,01 Gew.-%, enthalten sein, wobei sich die Mengenangaben auf das Gesamtgewicht des Mittels beziehen,
   - Vitamin B₇: (Biotin), auch als Vitamin H oder "Hautvitamin" bezeichnet. Bei Biotin handelt es sich um (3aS,4S, 6a*R*)-2-Oxohexahydrothienol[3,4-*d*]-imidazol-4-valeriansäure. Biotin kann in den erfindungsgemäß zu verwendenden Mitteln bevorzugt in Mengen von 0,0001 bis 1,0 Gew.-%, insbesondere in Mengen von 0,001 bis 0,01 Gew.-% enthalten sein, wobei sich die Mengenangaben auf das Gesamtgewicht des Mittels beziehen,
(ii) Vitamin C (Ascorbinsäure): kann in den erfindungsgemäß zu verwendenden Mitteln bevorzugt in Mengen von 0,1 bis 3 Gew.-%, bezogen auf das gesamte Mittel, eingesetzt werden. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein,
(iii) Vitamin E: Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate können in den erfindungsgemäß zu verwendenden Mitteln bevorzugt in Mengen von 0,05 - 1 Gew.-%, bezogen auf das gesamte Mittel, enthalten sein,
(iv) Vitamin F: Unter Vitamin F werden üblicherweise essentielle Fettsäuren, insbesondere Linolsäure, Linolensäure und Arachidonsäure, verstanden,
(v) Vitamin H: Vitamin H ist eine andere Bezeichnung für Biotin oder Vitamin B₇ (siehe oben),
(vi) Vitamin K: Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K kann in den erfindungsgemäß zu verwendenden Mitteln bevorzugt in Mengen von 0,0001 bis 1,0 Gew.-%, insbesondere 0,01 bis 0,5 Gew.-%, jeweils bezogen auf das gesamte Mittel, enthalten sein.

Panthenol, Pantolacton, Nicotinsäureamid, Pyridoxin, Pyridoxamin, Pyridoxal, Biotin, Ascorbylpalmitat, -acetat, Mg-Ascorbylphosphat, Na-Ascorbylphosphat, Natrium- und Magnesiumascorbat und die Tocopherolester, besonders Tocopherylacetat, Panthenol und Pantolacton sind besonders bevorzugt.

Geeignete Monomere von Aminosäuren und/oder der N-C₂-C₂₄-Acylaminosäuren können ausgewählt sein aus Alanin, Arginin, Asparagin, Asparaginsäure, Canavanin, Citrullin, Cystein, Cystin, Desmosin, Glutamin, Glutaminsäure, Glycin, Histidin, Hydroxylysin, Hydroxyprolin, Isodesmosin, Isoleucin, Leucin, Lysin, Methionin, Ornithin, Phenylalanin, Prolin, Pyroglutaminsäure, Sarcosin, Serin, Taurin, Threonin, Thyroxin, Tryptophan, Tyrosin und Valin. Besonders bevorzugt sind Taurin, Arginin, Hydroxyprolin, Prolin und Glutaminsäure.
Der C₂-C₂₄-Acylrest, mit dem die genannten Aminosäuren an der Aminogruppe derivatisiert sind, ist bevorzugt ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Bevorzugte Beispiele sind Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Zinkpyroglutamat und Natriumlauroylglutamat.
Physiologisch verträgliche Salze der zuvor genannten Wirkstoffe, die Säuregruppen enthalten und Salze bilden können, können ausgewählt sein aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.
Geeignete Oligomere von Aminosäuren und/oder der N-C₂-C₂₄-Acylaminosäuren können ausgewählt sein aus Di-, Tri-, Tetra-, Penta-, Hexa-, Hepta-, Octa-, Nona- und Decapeptiden, die acyliert und/oder verestert sein können. Besonders bevorzugt sind die Di-, Tri-, Tetra-, Penta- und Hexapeptide, die acyliert und/oder verestert sein können. Bevorzugte, gegebenenfalls acylierte und/oder veresterte Dipeptide sind Tyr-Arg, Val-Trp, Asn-Phe, Asp-Phe, N-Palmitoyl-ß-Ala-His, N-Acetyl-Tyr-Arg-hexyldecylester (z.B. Calmosensine von Sederma), Carnosin (ß-Ala-His) und N-Palmitoyl-Pro-Arg. Bevorzugte, gegebenenfalls acylierte und/oder veresterte Tripeptide sind Gly-His-Lys, N-Palmitoyl-Gly-His-Lys, Gly-Lys-His, His-Ala-Orn, Lys-Phe-Lys, N-Elaidoyl-Lys-Phe-Lys und N-Acetyl-Arg-Lys-Arg-NH₂. Bevorzugte, gegebenenfalls acylierte und/oder veresterte Tetrapeptide sind Gly-Gln-Pro-Arg, Gly-Gln-Arg-Pro und N-Palmitoyl-Gly-Gln-Pro-Arg. Bevorzugte, gegebenenfalls acylierte und/oder veresterte Pentapeptide sind Lys-Thr-Thr-Lys-Ser, N-Palmitoyl-Lys-Thr-Thr-Lys-Ser, N-Palmitoyl-Tyr-Gly-Gly-Phe-Met und N-Palmitoyl-Tyr-Gly-Gly-Phe-Leu. Ein bevorzugtes Hexapeptid ist Palmitoyl-Val-Gly-Val-Ala-Pro-Gly (Biopeptide EL von Sederma).
Es kann besonders bevorzugt sein, dass die erfindungsgemäß verwendeten Mittel ein Gemisch aus mindestens zwei Oligopeptiden enthalten. Ein besonders bevorzugtes Gemisch ist die Kombination aus N-Palmitoyl-Gly-His-Lys (z.B. Biopeptide CL von Sederma) und N-Palmitoyl-Gly-Gln-Pro-Arg (z.B. in Eyeliss von Sederma). Eine vorgefertigte Mischung des Tripeptids Palmitoyl-Gly-His-Lys und des Tetrapeptids N-Palmitoyl-Gly-Gln-Pro-Arg ist unter dem Handelsnamen Matrixyl 3000, ebenfalls von Sederma, erhältlich.

Geeignete Polymere von Aminosäuren und/oder der N-C₂-C₂₄-Acylaminosäuren können ausgewählt sein aus pflanzlichen und tierischen Proteinhydrolysaten und/oder Proteinen. Unter tierischen Proteinhydrolysaten sind z. B. Elastin-, Collagen-, Keratin-, Seiden- und Milcheiweiß-Proteinhydrolysate zu verstehen, die auch in Form von Salzen vorliegen können. Bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, z. B. die Handelsprodukte Phytokine^{®} von Coletica oder Ridulisse C^{®} von Silab.
Proteinhydrolysate können naturgemäß auch monomere Aminosäuren und Oligopeptide enthalten; ihre Zusammensetzung ist normalerweise nicht definiert.
Ebenfalls möglich ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).
Erfindungsgemäß einsetzbar sind auch kationisierte Proteinhydrolysate.
In einer weiteren bevorzugten Ausführungsform können die Polymeren der Aminosäuren ausgewählt sein aus DNA-Reparaturenzymen.
Bevorzugte DNA-Reparaturenzyme sind Photolyase und T4 Endonuclease V, letztere im Weiteren mit "T4N5" abgekürzt. Diese beiden Enzyme sind im Stand der Technik bereits als so genannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.

Besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.
In den erfindungsgemäß zu verwendenden Mitteln können die Photosome™ oder Ultrasome™ bevorzugt in Mengen von 0,1 - 10 Gew.-%, mehr bevorzugt von 0,5 - 5,0 Gew.-% und besonders bevorzugt von 1,0 - 4,0 Gew.-%, bezogen auf das gesamte Mittel, enthalten sein.
In den erfindungsgemäß zu verwendenden Mitteln können die Monomere, Oligomere oder Polymere von Aminosäuren, N-C₂-C₂₄-Acylaminosäuren und/oder den Estern und/oder den physiologisch verträglichen Metallsalzen dieser Substanzen bevorzugt in Mengen von 0,0001 - 10 Gew.-%, mehr bevorzugt von 0,01 - 5 Gew.-% und besonders bevorzugt von 0,1 - 3 Gew.-%, jeweils bezogen auf das gesamte Mittel, enthalten sein.

Unter geeigneten DNA- oder RNA-Oligonucleotiden werden bevorzugt Polymerisate aus 2 bis 20, mehr bevorzugt aus 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5'-triphosphat) und GTP (Guanosin-5'-triphosphat).
Ein besonders bevorzugtes Oligonucleotid ist das Thymidin-Dinucleotid.
In den erfindungsgemäß zu verwendenden Mitteln können die DNA-Oligonucleotide oder RNA-Oligonucleotide vorzugsweise in Mengen von 0,0001 - 5 Gew.-%, bevorzugt von 0,001 - 1,0 Gew.-% und besonders bevorzugt von 0,01 - 0,5 Gew.-%, bezogen auf das gesamte Mittel, enthalten sein.

Geeignete natürliche Betainverbindungen sind bevorzugt natürlich vorkommende Verbindungen mit der Atomgruppierung R₃N⁺-CH₂-X-COO⁻ gemäß IUPAC-Regel C-816.1. So genannte Betaintenside (synthetisch) fallen nicht unter die geeigneten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).
Die Betainverbindungen können in den erfindungsgemäß zu verwendenden Mitteln vorzugsweise in einer Gesamtmenge von 0,05 bis 5 Gew.-%, bevorzugt 0,1 bis 3 Gew.-%, besonders bevorzugt 0,5 bis 2 Gew.-%, jeweils bezogen auf das gesamte Mittel, enthalten sein.

Unter geeigneten α-Hydroxycarbonsäuren, α-Ketocarbonsäuren oder β-Hydroxycarbonsäuren oder deren Ester-, Lacton- oder Salzformen werden bevorzugt Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxy-dodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure verstanden. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren können ausgewählt sein aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren, α-Ketocarbonsäuren oder β-Hydroxycarbonsäuren oder ihre Derivate können in den erfindungsgemäß zu verwendenden Mitteln vorzugsweise in Mengen von 0,1 - 10 Gew.-%, bevorzugt von 0,5 - 5 Gew.-% enthalten sein, wobei sich die Mengenangaben auf das gesamte Mittel beziehen.

Bevorzugte Flavonoide umfassen die Glycoside der Flavone, der Flavanone, der 3-Hydroxyflavone (Flavonole), der Aurone und der Isoflavone. Besonders bevorzugte Flavonoide können ausgewählt sein aus Naringin (Aurantiin, Naringenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).
Außerordentlich bevorzugte Flavonoide sind α-Glucosylrutin, Naringin und Apigenin-7-glucosid. Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.
Die Flavonoide können in den erfindungsgemäß verwendeten Mitteln vorzugsweise in Mengen von 0,0001 bis 1 Gew.-%, bevorzugt von 0,0005 bis 0,5 Gew.-% und besonders bevorzugt von 0,001 bis 0,1 Gew.-% enthalten sein, jeweils bezogen auf die Flavonoidaktivsubstanz in dem gesamten kosmetischen Mittel.
Zu den geeigneten Isoflavonoiden werden bevorzugt die Isoflavone und die Isoflavon-Glycoside gezählt.
Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.
In bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht.

Besonders bevorzugte Isoflavon-Glycoside sind Daidzin und Genistin.

Weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt können Isoflavone oder Isoflavon-Glycoside in Form von aus Soja gewonnenen Extrakten in den erfindungsgemäß verwendeten Mitteln eingesetzt werden, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoidreicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Die Isoflavonoide können in den erfindungsgemäß verwendeten Mitteln vorzugsweise in Mengen von 0,00001 bis 1 Gew.-%, bevorzugt von 0,0005 bis 0,5 Gew.-% und besonders bevorzugt von 0,001 bis 0,1 Gew.-% enthalten sein, jeweils bezogen auf die Isoflavonoidaktivsubstanz in dem gesamten kosmetischen Mittel.

In einer weiteren bevorzugten Ausführungsform können die erfindungsgemäß zu verwendenden Mittel zusätzlich mindestens ein Polyphenol oder einen Polyphenol-reichen Pflanzenextrakt enthalten.
Unter Polyphenolen sind bevorzugt aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.
Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kernen der Chardonnay-Traube, erhältlich von der Firma Croda.
Die Polyphenole können in den erfindungsgemäß zu verwendenden Mitteln vorzugsweise in Mengen von 0,001 bis 10 Gew.-%, bevorzugt von 0,005 bis 5 Gew.-% und besonders bevorzugt von 0,01 bis 3 Gew.-%, jeweils bezogen auf das gesamte kosmetische Mittel, enthalten sein.

In einer weiteren bevorzugten Ausführungsform können die erfindungsgemäß verwendeten Mittel weiterhin mindestens ein Ubichinon oder ein Ubichinol oder deren Derivate enthalten. Ubichinole sind die reduzierte Form der Ubichinone. Bevorzugte Ubichinone weisen die Formel (III) auf: 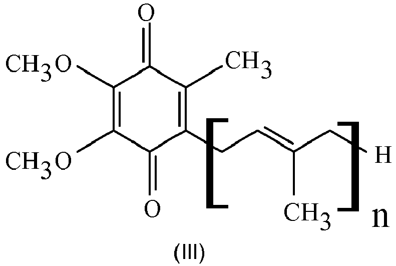 mit n = 6, 7, 8, 9 oder 10.
Besonders bevorzugt ist das Ubichinon der Formel (III) mit n = 10, auch bekannt als Coenzym Q10.
Die Ubichinone, Ubichinole oder deren Derivate können in den erfindungsgemäß zu verwendenden Mitteln vorzugsweise in Mengen von 0,0001 bis 1 Gew.-%, bevorzugt von 0,001 bis 0,5 Gew.-% und besonders bevorzugt von 0,005 bis 0,1 Gew.-%, jeweils bezogen auf das gesamte Mitte, enthalten sein.

Silymarin stellt ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Silymarin kann in den erfindungsgemäß zu verwendenden Mitteln vorzugsweise in Mengen von 0,0001 bis 1 Gew.-%, bevorzugt von 0,001 bis 1,0 Gew.-% und besonders bevorzugt von 0,005 bis 0,5 Gew.- %, jeweils bezogen auf das gesamte Mittel, enthalten sein.

In einer weiteren bevorzugten Ausführungsform können die erfindungsgemäß zu verwendenden Mittel mindestens ein natürlich vorkommendes Xanthin-Derivat, ausgewählt aus Coffein, Theophyllin, Theobromin und Aminophyllin, enthalten. Besonders bevorzugt ist Coffein.

Die natürlich vorkommenden Xanthin-Derivate können in den erfindungsgemäß zu verwendenden Mitteln vorzugsweise in Mengen von 0,0001 bis 5 Gew.-%, bevorzugt von 0,001 bis 3 Gew.-% und besonders bevorzugt von 0,005 bis 1 Gew.-%, jeweils bezogen auf das gesamte Mittel, enthalten sein.

Ectoin ist der Trivialname für 2-Methyl-1,4,5,6-tetrahydropyrimidin-4-carboxylat. Ectoin kann in den erfindungsgemäß zu verwendenden Mitteln vorzugsweise in Mengen von 0,0001 bis 1 Gew.-%, bevorzugt von 0,001 bis 0,5 Gew.-% und besonders bevorzugt von 0,005 bis 0,01 Gew.-%, jeweils bezogen auf das gesamte Mittel, enthalten sein.

Bei geeigneten UV-Filtersubstanzen handelt es sich bevorzugt um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UVA-Filter und UVB-Filter. Die UVA- und UVB-Filter können sowohl einzeln als auch in Mischungen eingesetzt werden. Der Einsatz von Filter-Mischungen ist bevorzugt.
Besonders geeignete UV-Filter können ausgewählt sein aus den physiologisch verträglichen Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethyl-hexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxy-zimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone, Uvinul^{®} T 150), Dimethicodiethylbenzal malonate (CAS-Nr. 207574-74-1, Parsol^{®} SLX), Dioctyl Butamido Triazone (Uvasorb^{®} HEB), 2,4-bis-[5-1(di- methylpropyl)benzoxazol-2-yl-(4-phenyl)-imino]-6-(2-ethylhexyl)-imino-1,3,5-triazin (CAS Nr. 288254-16-0, Uvasorb^{®} K2A) und sowie beliebige Mischungen der genannten Komponenten.
Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexyl-salicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bevorzugte anorganische Lichtschutzpigmente sind feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.
Die organischen UV-Filtersubstanzen können in den erfindungsgemäß zu verwendenden Mitteln bevorzugt in Mengen von 0,01 - 30 Gew.-%, mehr bevorzugt von 0,05 - 20 Gew.-%, besonders bevorzugt von 0,1 - 15 Gew.-% und außerordentlich bevorzugt von 0,25 - 10 Gew.-%, jeweils bezogen auf das gesamte Mittel, enthalten sein.
Die anorganischen UV-Filtersubstanzen können in den erfindungsgemäß zu verwendenden Mitteln vorzugsweise in Mengen von 0,01 - 15 Gew.-%, bevorzugt von 0,05 - 10 Gew.-%, besonders bevorzugt von 0,1 - 5 Gew.-% und außerordentlich bevorzugt von 0,25 - 4,0 Gew.-%, jeweils bezogen auf das gesamte Mittel, enthalten sein.

Geeignete hautberuhigende Wirkstoffe sind bevorzugt ausgewählt aus Farnesol, Allantoin, α-Bisabolol und α-Liponsäure, die in den erfindungsgemäß zu verwendenden Mitteln vorzugsweise in Mengen von 0,001 bis 5 Gew.-%, bevorzugt von 0,005 bis 1 Gew.-%, jeweils bezogen auf das gesamte Mittel, enthalten sein.

Geeignete sebumregulierende Wirkstoffe können ausgewählt sein aus Acnacidol, Azelainsäure, Azeloglycina und einem Extrakt aus Spiraea Ulmaria. Der Extrakt ist z.B. im Produkt Seburegul der Firma Silab enthalten. Die sebumregulierenden Wirkstoffe können in den erfindungsgemäß zu verwendenden Mitteln vorzugsweise in Mengen von 0,001 bis 5 Gew.-%, bevorzugt 0,01 bis 2 Gew.-% und besonders bevorzugt 0,1 bis 1 Gew.-%, jeweils bezogen auf das gesamte Mittel, enthalten sein.

In einer besonders bevorzugten Ausführungsform wird der mindestens eine wässrige oder wässrig-alkoholische Pflanzenextrakt in kosmetischen Hautbehandlungsmitteln verwendet, die neben dem zuvor beschriebenen Träger bevorzugt mindestens zwei, mehr bevorzugt mindestens drei und insbesondere bevorzugt mindestens vier Wirkstoffe aus der zuvor genannten Gruppe enthalten. Insbesondere bevorzugt ist die erfindungsgemäße Verwendung in Hautbehandlungsmitteln, die als weitere Wirkstoffe mindestens zwei Wirkstoffe aus der Gruppe der Vitamine (wie zuvor beschrieben), der Harnstoff-Derivate nach Formel (I), der natürlichen Betainverbindungen, der Xanthin-Derivate und/oder der hautberuhigenden Wirkstoffe enthalten.

Erfindungsgemäß zu verwendende Mittel können auch als Pasten, Salben, Lotionen oder Cremes vorliegen. Feste Mittel können beispielsweise als loser Puder, gepresster Puder oder als Stift vorliegen.

Vorteilhafterweise liegen die erfindungsgemäß zu verwendende Hautbehandlungsmittel in Form einer flüssigen, fliessfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

Besonders bevorzugte erfindungsgemäß zu verwendende Mittel liegen in Form einer fließfähigen Emulsion vor und enthalten bevorzugt mindestens einen weiteren hautkonditionierenden Wirkstoff und mindestens einen Emulgator.

Unter geeigneten konditionierenden Wirkstoffen sind bevorzugt solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen. Bevorzugte konditionierende Wirkstoffe können ausgewählt sein aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsaeuren, besonders linearen und/oder verzweigten, gesaettigten und/oder ungesaettigten C₈₋₃₀-Fettsaeuren, Fettalkoholen, besonders gesättigten, ein- oder mehrfach ungesättigten, verzweigten oder unverzweigten Fettalkoholen mit 4-30 Kohlenstoffatomen, die mit 1-75, bevorzugt 5-20 Ethylenoxid-Einheiten ethoxyliert und/oder mit 3-30, bevorzugt 9-14 Propylenoxid-Einheiten propoxyliert sein können, Esterölen, das heißt Estern von C₆₋₃₀-Fettsaeuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsaeureestern von gesättigten und/oder ungesättigten linearen und/oder verzweigten Fettsäuren mit Glycerin, die mit 1-10, bevorzugt 7- 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesaettigter und gegebenenfalls hydroxylierter C₆₋₃₀-Fettsaeuren, z. B. gehärteten Triglyceridfetten, Phospholipiden, beispielsweise Sojalecithin, Ei-Lecithin und Kephalinen, Shea-Butter, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin-und/oder Hydroxy-Gruppen enthalten, bevorzugt Substanzen mit den INCI-Bezeichnungen Dimethiconol, Amodimethicone oder Trimethylsilylamodimethicone. Die Einsatzmenge der Fettstoffe in den erfindungsgemäß zu verwendenden Mitteln beträgt bevorzugt 0,1-99 Gew.-%, besonders bevorzugt 2-50 Gew.-% und außerordentlich bevorzugt 5-20 Gew.-%, jeweils bezogen auf das gesamte Mittel.

Geeignete oberflächenaktive Substanzen und/oder Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsaeuren und an C₈-C₁₅-Alkylphenole, C₁₂-C₂₂-Fettsaeuremono- und - diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®}68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsaeuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsaeureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsaeuren und deren Na-, K-, Ammonium-, Ca-, Mg-und Zn-Salze.

Die erfindungsgemäß zu verwendenden Mittel können die Emulgatoren bevorzugt in Mengen von 0,1 bis 25 Gew.-%, besonders bevorzugt 0,5-15 Gew.-%, bezogen auf das gesamte Mittel, enthalten.

Weitere geeignete Zusatzstoffe der Emulsionen sind Verdickungsmittel, z. B. anionische Polymere aus Acrylsäure, Methacrylsäure, Crotonsäure, Maleinsäureanhydrid und 2-Acrylamido-2-methylpropansulfonsaeure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäeure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®} 600, Simulgel^{®} NS und Simulgel^{®} EPG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Tego Carbomer^{®} oder Carbopol^{®}. Ein besonders bevorzugtes anionisches Copolymer enthält als Monomer zu 80-98% eine ungesättigte, gewünschtenfalls substituierte C₃₋₆-Carbonsaeure oder ihr Anhydrid sowie zu 2-20% gewünschtenfalls substituierte Acrylsäureester von gesättigten C₁₀₋₃₀-Carbonsaeuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Weitere geeignete Zusatzstoffe sind beispielsweise:
- Parfumöle,
- Substanzen zur Einstellung des pH-Wertes,
- Komplexbildner wie EDTA, NTA, β-Alanindiessigsaeure und Phosphonsaeuren,
- Treibmittel wie Propan-Butan-Gemische, Pentan, Isopentan, Isobutan, N₂O, Dimethylether, CO₂ und Luft,
- Wirkstoffe wie Hyaluronsäure und/oder Glycyrrhizinsäure bzw. deren physiologisch verträglich Salze und/oder Derivate,
- Pigmente
- Abrasivstoffe und/oder
- Konservierungsmittel.

Ein zweiter Gegenstand der Erfindung ist ein kosmetisches, nichttherapeutisches Verfahren zur optischen Verbesserung und/oder Erhaltung des Erscheinungsbildes menschlicher Haut, bei dem zur Inaktivierung und/oder Eliminierung von Milben eine kosmetische Zusammensetzung, enthaltend mindestens einen wässrigen oder wässrig-alkoholischen Pflanzenextrakt, auf die Haut aufgetragen und nach einer Einwirkungszeit von 30 Sekunden bis 24 Stunden gegebenenfalls mit Wasser wieder abgespült wird.

In einer bevorzugten Ausführungsform des zweiten Erfindungsgegenstandes ist ein Verfahren besonders bevorzugt, bei dem die kosmetische Zusammensetzung eine Mischung aus
- mindestens einem wässrigen oder wässrig-alkoholischen Extrakt aus den Wurzeln von *Zingiber officinalis,*
- mindestens einem wässrigen oder wässrig-alkoholischen Extrakt aus der Rinde von *Cinnamomum Cassia,* und
- mindestens einem wässrigen oder wässrig-alkoholischen Pflanzenextrakt aus den Wurzeln von *Sanguisorba officinalis* enthält.

Bezüglich bevorzugter Ausführungsformen des erfindungsgemäßen Verfahrens gilt mutatis mutandis das zu der erfindungsgemäßen Verwendung Gesagte.

Die erfindungsgemäße Verwendung einer mindestens einen wässrigen oder wässrig-alkoholischen Pflanzenextrakt enthaltenden kosmetischen Zubereitung führt zu einer gründlichen und nachhaltigen Inaktivierung oder Eliminierung von Hautmilben.
Der Ausbruch einer Demodikose kann durch die erfindungsgemäße Verwendung verhindert werden.

Ein weiterer Vorteil ist, dass sich die erfindungsgemäße Verwendung in leave-on- und in rinse-off-Produkten eignet, und demnach bei jeder beliebigen kosmetischen Behandlung zum Einsatz kommen kann.
Die Pflanzenextrakte sind natürlichen Ursprungs und belasten die Haut nicht.
Schließlich kann die Verwendung einen wässrigen oder wässrig-alkoholischen Pflanzenextrakt enthaltender kosmetischer Zubereitung zur nachhaltigen Inaktivierung oder Eliminierung von Hautmilben auch unterstützend bei der Anwendung anderer Produkte genutzt werden, beispielsweise bei der kosmetischen Behandlung von Neurodermitis, Ekzemen, Psoriasis, Rosacea, Ulcus etc..

Die nachfolgenden Beispiele sollen die vorliegende Erfindung verdeutlichen, ohne sie auf darauf einzuschränken.

### Beispiele:

### 1) Die Pflanzenextraktmischung Sebustop^{®} (Solabia) wurde auf ihre Wirksamkeit in Bezug auf die Inaktivierung und/oder Eliminierung von Hautmilben wie folgt getestet:

Die zu testende Substanz wurde mit Wasser auf die vorgesehene Einsatzkonzentration verdünnt (s.u.). Haustaubmilben, die aufgrund ihrer großen Ähnlichkeit als Vergleich zu Haarbalgmilben (Demodex) verwendet werden, wurden aus ihrem Futter isoliert und auf 1,5 cm x 1,5 cm große Filterpapierstücke übertragen. Etwa 100 Milben je Ansatz wurden mit 50 µl der zu testenden Substanz in den vorhergesehenen Verdünnungen betropft. Die überschüssige Flüssigkeit wurde nach 10 Sekunden abgesaugt. Die noch feuchten Filterpapiere mit den Milben wurden nach der Behandlung in eine Petrischale gebracht, die mit einem Deckel und einer Umrandung mit Paraffin verschlossen wurden, um sicherzustellen, dass evtl. überlebende Milben nicht entweichen können. Die Milben können nach Beginn einer Behandlung - gleichgültig mit welchem Mittel - unbeweglich werden, sich jedoch später wieder erholen und ihre Beweglichkeit zumindest für kurze Zeit wiedererlangen. Andererseits ist es auch möglich, dass Milben in den ersten Stunden nach einer Behandlung noch vital erscheinen, jedoch bereits so stark geschädigt sind, dass sie innerhalb eines Tages bis zu 2-3 Tagen sterben. Daher wurden die Petrischalen für einen Tag aufbewahrt, und die Milben dann erst auf ihre Vitalität bewertet. Die Anzahl der toten und lebenden Milben wurde bestimmt und in % der Sterberate umgerechnet.
Die Versuche wurden zweifach durchgeführt. Als Kontrolle dienten Versuche mit Leitungswasser.
Sebustop^{®} (INCI: Butylene Glycol, Water, Poterium (Sanguisorba) officinale root extract, Cinnamonum cassia bark extract and Zingiber officinale root extract) in einer Einsatzkonzentration von 4%:
Gestorbene Milben in %:
   Test 1: 27,78
   Test 2: 10,99
   Sterberate (Mittelwert): 19,4%

Das Ergebnis zeigt deutlich, dass Sebustop^{®} wirksam gegen die Inaktivierung bzw. Eliminierung von Milben ist.

### 2) Ausführungsbeispiele kosmetischer leave-on-Zubereitungen (Mengenangaben in [Gew.-%]):

| | **Beispiel 1** | **Beispiel 2** | **Beispiel 3** |
|---|---|---|---|
| **Montanov 202^{®1}** | 5,00 | 5,00 | 5,00 |
| **Caprylic/Capric Triglyceride** | 10,00 | 10,00 | 10,00 |
| **Cetiol CC^{®2}** | 3,00 | 3,00 | 3,00 |
| **Cocosglyceride C12-18** | 2,00 | 2,00 | 2,00 |
| **Vitamin E Acetate** | 0,50 | 0,50 | 0,50 |
| **Controx KS C^{®3}** | 0,05 | 0,05 | 0,05 |
| **Cetiol SB^{®4} 45** | 1,50 | 1,50 | 1,50 |
| **DC 8040^{®5}** | 0,90 | 0,90 | 0,90 |
| **Glycerin (99,5%)** | 5,00 | 5,00 | 5,00 |
| **Hexanediol-1,6** | 6,00 | 6,00 | 6,00 |
| **Tego Carbomer 140^{®6}** | 0,40 | 0,40 | 0,40 |
| **TiO₂** | 0,20 | 0,20 | 0,20 |
| **Betafin BP 20^{®7}** | 2,00 | 2,00 | 2,00 |
| **Ridulisse C GR^{®8}** | 0,50 | 0,50 | 0,50 |
| **DSC-H^{®9}** | 2,00 | 2,00 | 2,00 |
| **Phenonip^{®10}** | 0,50 | 0,50 | 0,50 |
| **Sebustop^{®11}** | 2,00 | 3,00 | 4,00 |
| **Allantoin** | 0,05 | 0,05 | 0,05 |
| **D-Panthenol** | 0,75 | 0,75 | 0,75 |
| **Dragosantol 100^{®12}** | 0,05 | 0,05 | 0,05 |
| **Matrixyl 3000^{®13}** | 3,00 | 3,00 | 3,00 |
| **Simulgel EPG^{®14}** | 1,00 | 1,00 | 1,00 |
| **NaOH, Parfum, Farbstoff** | q.s. | q.s. | q.s. |
| **Wasser** | ad 100 | ad 100 | ad 100 |

In den zuvor genannten Beispielen wurden die folgenden Handelsprodukte verwendet:
1 INCI-Bezeichnung: Arachidyl Alcohol, Behenyl Alcohol, Arachidyl Glucoside; Seppic
2 INCI-Bezeichnung: Dicaprylyl Carbonate; BASF
3 INCI-Bezeichnung: Tocopherol, Hydrogenated Palm Glycerides Citrate; BASF
4 INCI-Bezeichnung: Shea Butter; BASF
5 INCI-Bezeichnung: Isododecane, Dimethicone Crosspolymer; Dow Corning
6 INCI-Bezeichnung: Carbomer; Evonik
7 INCI-Bezeichnung: Betaine (Trimethylglycin); Finnfeeds Finnland Oy
8 INCI-Bezeichnung: Hydrolyzed Soy Protein; Silab
9 INCI-Bezeichnung: Dimethylsilanol Hyaluronate; Exsymol
10 INCI-Bezeichnung: Phenoxyethanol, Methylparaben, Ethylparaben; Clariant
11 INCI-Bezeichnung: Butylene Glycol, Water, Poterium (Sanguisorba) officinale root extract, Cinnamonum cassia bark extract and Zingiber officinale root extract); Solabia
12 INCI-Bezeichnung: Bisabolol; Symrise
13 INCI-Bezeichnung: Glycerin, Aqua (Water), Butylene Glycol, Carbomer, Coco Glucoside, Palmitoyl Oligopeptide, Palmitoyl Tetrapeptide-7; Sederma
14 INCI-Bezeichnung: Sodium Acrylate/Sodium Acryloyldimethyl taurate Copolymer, Polyisobutene, Caprylyl/Capryl Glucoside; Seppic

## Patentansprüche

1. Kosmetische Verwendung einer kosmetischen Zusammensetzung, die mindestens einen wässrigen oder wässrig-alkoholischen Pflanzenextrakt enthält, für die Inaktivierung und/oder Eliminierung von Milben auf der Haut.

2. Kosmetische Verwendung nach Anspruch 1 für die Inaktivierung und/oder Eliminierung von Milben auf der menschlichen Gesichts- und/oder Kopfhaut.

3. Kosmetische Verwendung nach einem der Ansprüche 1 oder 2 für die Inaktivierung und/oder Eliminierung von Milben aus der Gattung der Haarbalgmilben *(Demodex).*

4. Kosmetische Verwendung nach einem der Ansprüche 1 bis 3 für die Inaktivierung und/oder Eliminierung von Milben der Art *Demodex folliculorum.*

5. Kosmetische Verwendung nach einem der Ansprüche 1 bis 4, **dadurch gekennzeichnet, dass** die kosmetische Zusammensetzung eine Mischung aus
- mindestens einem wässrigen oder wässrig-alkoholischen Pflanzenextrakt aus der Familie der Ingwergewächse und/oder
- mindestens einem wässrigen oder wässrig-alkoholischen Pflanzenextrakt aus der Familie der Lorbeergewächse und/oder
- mindestens einem wässrigen oder wässrig-alkoholischen Pflanzenextrakt aus der Familie der Rosengewächse enthält.

6. Kosmetische Verwendung nach Anspruche 5, **dadurch gekennzeichnet, dass** die kosmetische Zusammensetzung eine Mischung aus
- mindestens einem wässrigen oder wässrig-alkoholischen Extrakt aus den Wurzeln von *Zingiber officinalis,*
- mindestens einem wässrigen oder wässrig-alkoholischen Extrakt aus der Rinde von *Cinnamomum Cassia,* und
- mindestens einem wässrigen oder wässrig-alkoholischen Pflanzenextrakt aus den Wurzeln von *Sanguisorba officinalis* enthält.

7. Kosmetische Verwendung nach einem der Ansprüche 1 bis 6, **dadurch gekennzeichnet, dass** der oder die wässrige(n) oder wässrig-alkoholische(n) Pflanzenextrakt(e) in der kosmetischen Zusammensetzung in einer Gesamtmenge von 0,1 bis 40 Gew.-%, bevorzugt von 1 bis 20 Gew.-% und insbesondere von 1,5 bis 5 Gew.-% enthalten ist (sind), wobei sich die Mengen auf das Gesamtgewicht der kosmetischen Zusammensetzung beziehen.

8. Kosmetische Verwendung nach einem der Ansprüche 1 bis 7, **dadurch gekennzeichnet, dass** die kosmetische Zusammensetzung als leave-on oder rinse-off-Zusammensetzung auf der Haut angewendet wird.

9. Kosmetisches, nichttherapeutisches Verfahren zur optischen Verbesserung und/oder Erhaltung des Erscheinungsbildes menschlicher Haut, **dadurch gekennzeichnet, dass** zur Inaktivierung und/oder Eliminierung von Milben eine kosmetische Zusammensetzung, enthaltend mindestens einen wässrigen oder wässrig-alkoholischen Pflanzenextrakt, auf die Haut aufgetragen und nach einer Einwirkungszeit von 30 Sekunden bis 24 Stunden gegebenenfalls mit Wasser wieder abgespült wird.

10. Kosmetisches, nichttherapeutisches Verfahren nach Anspruch 9, **dadurch gekennzeichnet, dass** die kosmetische Zusammensetzung eine Mischung aus
- mindestens einem wässrigen oder wässrig-alkoholischen Extrakt aus den Wurzeln von *Zingiber officinalis,*
- mindestens einem wässrigen oder wässrig-alkoholischen Extrakt aus der Rinde von *Cinnamomum Cassia,* und
- mindestens einem wässrigen oder wässrig-alkoholischen Pflanzenextrakt aus den Wurzeln von *Sanguisorba officinalis* enthält.
